# EUROPÄISCHE PATENTSCHRIFT

(11) **EP 1 637 233 B1**
(45) Veröffentlichungstag und Bekanntmachung des Hinweises auf die Patenterteilung: **03.02.2010**
(21) Anmeldenummer: 05027267.3
(22) Anmeldetag: 15.09.2000
(51) Int. Cl.: B05B 11/02, A61M 15/00, A61M 11/02

(54) **Vorrichtung zum ggf. zerstäubten Ausbringen eines insbesondere flüssigen Mediums**
Apparatus for dispensing, in particular for spraying fluid
Dispositif pour délivrer, notamment pour pulvériser un fluide

(30) Priorität: 15.09.1999 DE 19944211
(43) Veröffentlichungstag der Anmeldung: 22.03.2006
(62) Teilanmeldung aus: 00120051.8
(73) Patentinhaber: Ing. Erich Pfeiffer GmbH, 78315 Radolfzell (DE)
(72) Erfinder: Greiner-Perth, Jürgen, 78244 Gottmadingen (DE); Merk, Hans, 78343 Gaienhofen-Horn (DE)
(74) Vertreter: Patentanwälte Ruff, Wilhelm, Beier, Dauster & Partner

(56) Entgegenhaltungen:
- EP-A- 0 021 123
- EP-A- 0 452 728
- EP-A- 0 711 571
- US-A- 5 501 373

## Beschreibung

### Anwendungsgebiet und Stand der Technik

Aus der FR 2 625 981 A1 ist eine Vorrichtung zum ggf. zerstäubten Ausbringen eines insbesondere flüssigen Mediums bekannt, bei dem das Medium in einem Spritzen- oder Pumpenzylinder aufbewahrt ist und das Medium über einen Ausbringungskanal ausgebracht werden kann. Hierzu ist im Spritzenzylinder ein Kolben angeordnet, der von einem Stößel beaufschlagbar ist, wobei der Kolben manuell betätigbar ist. Die manuelle Betätigung des Stößels erfolgt dabei über einen Betätigungsdrücker, der in einem Gehäuse geführt ist, das auch den Spritzenzylinder aufnimmt. Dabei sind an dem Gehäuse und an dem Betätigungsdrücker Führungsmittel vorgesehen, die die Betätigung des Betätigungsdrückers in der axialen Richtung des Spritzenzylinders begrenzen, wodurch der Gesamthub des Stößels im Spritzenzylinder in mehrere definierte Teilhübe unterteilt wird. Um am Ende eines Teilhubes den nächsten Teilhub ausführen zu können, ist es erforderlich, den Betätigungsdrücker gegenüber dem Spritzenzylinder bzw. gegenüber dem Gehäuse in dem der Spritzenzylinder angeordnet ist, zu verdrehen.

Daneben ist aus der EP 0 334 349 A1 eine Vorrichtung zum Ausbringen eines Mediums bekannt, bei der der Stößel eines Spritzen- oder Pumpenzylinders über einen Betätigungsdrücker betätigt wird. Der Betätigungsdrücker wird dabei in einem Gehäuse geführt, das als Aufnahme für den Spritzenzylinder dient. Dabei sind an dem Gehäuse Führungsnocken ausgebildet, die in einer am Betätigungsdrücker ausgeformten Kulissenbahn geführt werden. Die Kulissenbahn ist so geformt, daß der Betätigungsweg des Betätigungsdrückers in mehrere Teilhübe unterteilt wird. Dies geschieht entweder dadurch, daß die Kulissenbahn treppenförmig verläuft, so daß zwischen zwei Teilhüben des Betätigungsdrückers ein Verdrehen des Betätigungsdrückers gegenüber dem Gehäuse erforderlich ist oder aber indem die Kulissenbahn überdrückbare Raststellen aufweist, die die in der Kulissenbahn geführte Nocke in einer Endlage eines Teilhubes so lange halten, bis die Betätigungskraft eine Haltekraft übersteigt. In dem letzteren Falle kann die Kulissenbahn auch linear ausgebildet sein.

Bei Vorrichtungen zum Ausbringen von Medien, bei denen ein Verdrehen des Betätigungsdrückers bzgl. dem Gehäuse erforderlich ist, um aufeinanderfolgende Teilhübe durchzuführen, ist es nachteilig, daß die Möglichkeit einer Einhandbetätigung nicht besteht. Zum Verdrehen des Betätigungsdrückers bzgl. dem Gehäuse ist es erforderlich, mit beiden Händen die Vorrichtung zu halten. Jedoch haben die gestuften Führungen den Vorteil, daß ein versehentliches aneinandergereihtes Durchführen zweier Teilhübe in einer Bewegung nicht möglich ist. Die Ausführung mit einer linearen Kulissenbahn und durch Überwindung einer Haltekraft überdrückbare Raststellen zur Unterteilung der Ausbringung des Mediums in mehrere Teilhübe, eröffnet zwar die Möglichkeit der Einhandbetätigung, es besteht hier aber die Gefahr, daß unbeabsichtigt mehrere Teilhübe in ununterbrochener Betätigung hintereinander ausgeführt werden.

Die EP 0 711 571 A1 zeigt eine Zerstäuberpumpe, bei der eine flexible, in Stufen kollabierende Haube einzelne Teilhübe voneinander abgrenzt.

Bei der US 5,501,373 A sorgen elastische Anschlagringe für eine Abgrenzung der Teilhübe.

Aus der EP 21 123 sind die im Oberbegriff des Anspruchs 1 genannten Merkmale bekannt.

### AUFGABE UND LÖSUNG

Aufgabe der Erfindung ist es, eine Vorrichtung zum Ausbringen eines flüssigen Mediums zu schaffen, bei der eine gleichmäßige Dosierung bei allen Teilhüben sichergestellt ist. Diese Aufgabe wird durch eine Vorrichtung nach Anspruch 1 gelöst.

Dadurch, daß die einzelnen Betätigungselemente unterschiedliche Betätigungswege aufweisen, können dennoch gleiche Teilchargen bei den aufeinanderfolgenden Teilbetätigungen erzielt werden. Insbesondere kann, um gleiche Ausbringungsvolumina sicherzustellen, der Betätigungsweg des ersten Teilhubes größer sein als der des zweiten Teilhubes, da gefunden wurde, daß während des ersten Teilhubes ein Leerweg zum Öffnen des Dichtelements 17 zu überwinden ist. Im im folgenden dargestellten Beispiel kann dies dadurch erreicht werden, daß eine Innenhülse um ein vorgegebenes Maß länger ist als der Betätigungsweg des Betätigungsdrückers beim ersten Teilhub.

Wenn vorteihaft ein Betätigungsdrücker an dem Stößel angreift, der den in dem Spritzen- oder Pumpenzylinder verschiebbaren Kolben betätigt, ist der Betätigungsweg des Betätigungsdrückers zwischen einer Betätigungsausgangs- und Betätigungsendlage so begrenzt, daß der daraus resultierende Ausbringungshub des Kolbens dem Maß entspricht, das für das Ausbringen einer Teilcharge benötigt wird.

Gemäß vorteilhaften Ausbildungen der Vorrichtung wird eine Rückstellfeder zum Zurückführen des Betätigungsdrückers aus der Betätigungsendlage in die Betätigungsausgangslage vorgesehen. An dem auf den Kolben des Spritzenzylinders einwirkenden Stößel sind Mitnehmer angeformt, die lediglich in Richtung des Ausbringungshubes eine kraftschlüssige Verbindung zwischen Betätigungsdrücker und Stößel herstellen. Zusätzlich können gemäß vorteilhafter Ausgestaltung an dem Stößel auch Rastmittel angeformt sein, die in Betätigungsausgangslage an einer Rastkante anliegen und die durch Überwinden einer Mindestbetätigungskraft des Betätigungsdrückers überdrückbar sind. Besonders vorteilhaft kann es sein, daß die Mitnehmer derart ausgebildet sind, daß die Mitnehmer eines Ausbringungshubes als Rastmittel eines nachfolgenden Ausbringungshubes dienen.

Gemäß einer Ausgestaltung der Erfindung sind die Mitnehmer als Verdickungen des Stößels ausgebildet, besonders vorteilhaft ist es, den Abschnitt des Stößels, der als Mitnehmer ausgebildet ist, als Kegelstumpf auszubilden, wobei die gedachte Spitze des Kegelstumpfes in Richtung des Kolbens des Spritzenzylinders liegt. Weitere vorteilhafte Ausgestaltungen der Vorrichtung zum Ausbringen eines Mediums können den weiteren Unteransprüchen entnommen werden.

Die vorstehenden und weitere Merkmale gehen außer aus den Ansprüchen auch aus der Beschreibung und den Zeichnungen hervor, wobei die einzelnen Merkmale jeweils für sich allein oder zu mehreren in Form von Unterkombinationen bei einer Ausführungsform der Erfindung und auf anderen Gebieten verwirklicht sein und vorteilhafte Ausführungen darstellen können. Die Unterteilung der Anmeldung in einzelne Abschnitte sowie Zwischen-Überschriften beschränken die unter diesen gemachten Aussagen nicht in ihrer Allgemeingültigkeit.

### KURZBESCHREIBUNG DER ZEICHNUNGEN

Ausführungsbeispiele der Erfindung sind in den Zeichnungen dargestellt und werden im folgenden näher erläutert. In den Zeichnungen zeigen:
- Fig. 1: in Schnittdarstellung ein erstes Ausführungsbeispiel einer Vorrichtung, bei der der Betätigungsweg des Betätigungsdrückers auf das für das Ausbringen einer Teilcharge benötigten Hub begrenzt ist und
- Fig. 2: den Querschnitt durch ein zweites Ausführungsbeispiel mit begrenztem Betätigungsweg eines Betätigungsdrückers.

### BESCHREIBUNG DER AUSFÜHRUNGSBEISPIELE

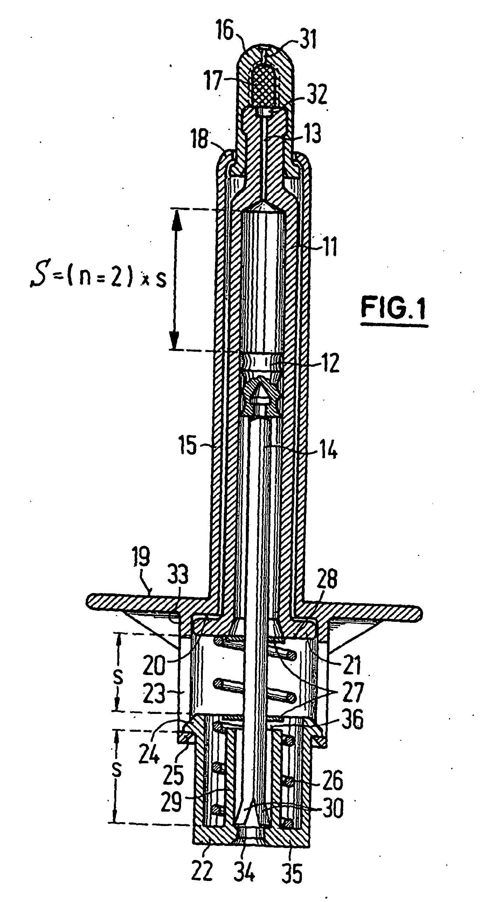

Die Fig. 1 zeigt den Querschnitt durch eine Vorrichtung zum ggf. zerstäubten Ausbringen eines insbesondere flüssigen Mediums. Das auszubringende Medium befindet sich in dem Spritzen- oder Pumpenzylinder 11, in dem der Kolben 12 in axialer Richtung des Spritzenzylinders verschiebbar gelagert ist. Der Spritzenzylinder 11 mündet in dem Ausbringungskanal 13, durch den hindurch das Medium ausgebracht wird, wenn über den Stößel 14 durch manuelle Betätigung nach Art einer Schubkolbenpumpe der Kolben 12 im Spritzenzylinder 11 in Richtung auf den Ausbringungskanal 13 hin verschoben wird.

Auf der Seite des Ausbringungskanals 13 wird über die Überwurfkante 18 des Gehäuses 15 eine Kappe 16 gehalten, in die der Ausbringungskanal 13 des Spritzenzylinders 11 mündet. Die Kappe 16 weist an ihrer dem Ausbringungskanal 13 abgewandten, vorderen Spitze eine Düse 31 als Austrittsöffnung für das auszubringende Medium auf. Zwischen Kappe 16 und Mündung 32 des Ausbringungskanals 13 befindet sich das Dichtelement 17. Das Dichtelement 17 stellt eine hermetische Abdichtung zwischen Düse 31 und Mündung 32 des Ausbringungskanals 13 zumindest solange dar, wie die Vorrichtung nicht zum ersten mal betätigt wurde. Dadurch wird zuverlässig eine Verkeimung oder ein Luftzutritt zu dem in dem Spritzenzylinder 11 aufbewahrten auszubringenden Medium sichergestellt. Dies ist besonders dann wichtig, wenn es sich bei dem auszubringenden Medium um ein steril aufzubewahrendes Arzneimittel handelt. Dies könnten beispielsweise Medikamente gegen Migräne oder aber Impfseren sein, die dem Patienten über die Nase auf die Nasenschleimhäute appliziert werden, da sie dort besonders gut vom Patienten absorbiert werden können.

Das Dichtelement 17 kann dabei entweder so ausgebildet sein, daß es bei der ersten Betätigung, also dem ersten Teilhub, irreversibel einen Kanal zwischen Mündung 32 des Ausbringungskanals 13 und der Düse 31 der Kappe 16 freigibt. Es ist aber auch möglich, das Dichtelement 17, beispielsweise aus einer elastischen Masse, so auszubilden, daß der Kanal zwischen Mündung 32 und Düse 31 sich jeweils nur für die Dauer des Teilhubes öffnet und anschließend sofort wieder verschließt. Letztere Ausbildung hat den Vorteil, daß weiterhin keine Luft über die Düse 31 zum Ausbringungskanal 13 gelangen kann.

Am dem Ausbringungskanal 13 gegenüberliegenden Ende weist der Spritzenzylinder 11 eine Aufweitung 21 auf, die sich an der Anlagefläche 20 des Gehäuses 15 abstützt. Die Anlagefläche 20 wird durch eine radiale Aufweitung des Gehäuses 15, die vorteilhaft gleichzeitig als Fingeranlage 19, zur Abstützung des Gehäuses 15 in der Hand des Benutzers während der Betätigung der Vorrichtung dient, gebildet.

Dadurch ist gleichzeitig sichergestellt, daß die Fingeranlage 19 schon vor dem ersten Teilhub - vom Ausbringungskanal 13 her gesehen - hinter dem Kolben 12 liegt, wodurch eine stabile und betätigungssichere Handhabung der Vorrichtung erreicht wird.

Die Aufweitung 21 des Spritzenzylinders 11 umgebend, ragt der Schaft 33 des Gehäuses 15 nach hinten ab. In dem Schaft 33 befinden sich Führungsschlitze 23, in die Rastnasen 24, die an dem Betätigungsdrücker 22 angeformt sind, eingreifen. Des weiteren weist der Schaft 33 an seinem hinteren Ende einen die Führungsschlitze 23 abschließenden Abschlußring 25 auf. Der Betätigungsdrücker 22 ist in seiner Außenkontur napfförmig ausgebildet und durch seine Rastnasen 24 in dem Schaft 33 axial verschiebbar gehalten. Der mögliche Betätigungsweg S des Betätigungsdrückers 22 zwischen seiner dargestellten Betätigungsausgangslage und der Betätigungsendlage wird durch die freie Länge der Führungsschlitze 23 in dem Schaft 33 des Gehäuses 15 festgelegt. In Betätigungsendlage liegt der Betätigungsdrücker 22 mit seiner Vorderkante an der Aufweitung 21 des Spritzenzylinders 11 an.

Zwischen der Aufweitung 21 des Spritzenzylinders 11 und dem Betätigungsdrücker 22 ist die Rückstellfeder 26 angeordnet. Die Rückstellfeder 26 hat zwei Funktionen. Durch ihre Vorspannung erzeugt sie eine zwischen Betätigungsdrücker 22 und Spritzenzylinder 11 wirkende Kraft, so daß einerseits der Betätigungsdrücker 22 des Gehäuses 15 in seiner Betätigungsausgangslage gehalten wird, andererseits wird auch der Spritzenzylinder 11 gegen axiales Verschieben im Gehäuse 15 gesichert.

Der Stößel 14 reicht vom Kolben 12 bis zu der Bodenfläche 35 des Betätigungsdrückers 22. An dem Stößel 14 sind Rastmittel 27 angeformt, die jeweils um den Teil-Betätigungsweg s voneinander beabstandet sind und die in Betätigungsausgangslage eines Teilhubes an der Rastkante 28, die vorzugsweise durch die Aufweitung 21 des Spritzenzylinders 11 gebildet wird, anliegen. Die Rastmittel 27 bilden im Zusammenwirken mit der Rastkante 28 einen der Betätigung des Betätigungsdrückers 22 entgegenwirkenden Druckpunkt, wodurch vorzugsweise sichergestellt wird, daß bei jeder Betätigung des Betätigungsdrückers 22 an ihm eine derartige Betätigungskraft aufgebracht wird, daß er immer in einer durchgehenden, ununterbrochenen Betätigung um seinen gesamten Betätigungsweg von der Betätigungsausgangslage in die Betätigungsendlage verbracht wird. An seinem, dem Kolben 12 abgewandten, hinteren Ende weist der Stößel 14 einen Mitnehmer 30 auf, der sich vor der ersten Betätigung in Anlage mit der Innenseite der Bodenfläche 35 befindet. Durch eine Einkerbung weist der Mitnehmer 30 eine radiale Vorspannung auf, die ihn radial in Richtung auf die Innenhülse 29, die an der Bodenfläche 35 des Betätigungsdrückers 22 angeformt ist und eine dem Betätigungsweg s entsprechende Länge aufweist, beaufschlagt.

Die Anzahl n der Teilhübe, in denen das im Spritzenzylinder 11 vorgehaltene Medium ausgebracht wird, beträgt bei der dargestellten Vorrichtung zwei. Somit ist auch das zur Verfügung stehende Volumen für das Medium im Spritzenzylinder vorgegeben. Das Volumen bestimmt sich aus dem Innendurchmesser des Spritzenzylinders 11 und der Verschiebelänge des Kolbens 12 zwischen seiner Ausgangslage (wie dargestellt) und seiner Endlage, wenn er um die vorgegebene Anzahl n von Teil-Betätigungswegen s, also um den gesamten Betätigungsweg S in Richtung auf den Ausbringungskanal 13 hin verschoben wurde. Es ist vorgesehen, daß die einzelnen Betätigungselemente unterschiedliche Betätigungswege aufweisen. Dies ist über den Abstand des Mitnehmers 30 zu der Bodenfläche 35 vor dem ersten und zu der Innenhülse 36 vor dem zweiten Betätigungshub möglich. Um gleiche Ausbringungsvolumina sicherzustellen ist der erste Teilhub größer als der zweite Teilhub, da während des ersten Teilhubes ein Leerweg zum Öffnen des Dichtelements 17 erforderlich ist. Im dargestellten Beispiel kann dies dadurch erreicht werden, daß die Innenhülse 29 um ein vorgegebenes Maß länger ist als der Betätigungsweg des Betätigungsdrückers 29 beim ersten Teilhub s.

Wird der Betätigungsdrücker 22 nunmehr betätigt, was in der Regel dadurch geschieht, daß der Betätigungsdrücker 22 auf der Außenseite der Bodenfläche 35 durch den Daumen des Benutzers mit einer Mindestkraft beaufschlagt wird, so wird der Betätigungsdrücker 22 um den Betätigungsweg s von der Betätigungsausgangslage - wie dargestellt - in die Betätigungsendlage verschoben. Über den Mitnehmer 30 wird der Stößel 14 beaufschlagt und um den gleichen Betätigungsweg s verschoben, wodurch in dem Spritzenzylinder 11 der Kolben 12 um den Betätigungsweg s verschoben wird. Ein dem dabei verdrängten Volumen entsprechendes Volumen an Medium wird aus dem Spritzenzylinder 11 durch den Ausbringungskanal 13 hindurch über die Kappe mit der Düse 31 hinaus ausgebracht und üblicherweise durch die Düse 31 zerstäubt. Zusätzlich zu ihrer Vorspannung wird dabei die Rückstellfeder 26 durch Verkürzung ihrer Länge weiter gespannt. Nach Beendigung der Betätigung wird der Betätigungsdrücker 22 losgelassen. Durch die zwischen Spritzenzylinder 11 und Betätigungsdrücker 22 aufgebrachte Kraft der Rückstellfeder 26 wird der Betätigungsdrücker 22 in die Betätigungsausgangslage zurück verbracht. Durch Formgebung des Kolbens 12 und durch die Wirkung der Rastmittel 27, die in den Spritzenzylinder 11 hineingedrückt wurden, kann sichergestellt werden, daß ein Zurückbewegen des Stößels 14 zusammen mit dem Betätigungsdrücker 22 nicht stattfindet. Vielmehr gleiten die Mitnehmer 30 entlang der Innenhülse 29. Sobald der Betätigungsdrücker 22 wieder die Betätigungsausgangslage, die durch den Abschlußring 25 definiert wird, erreicht, verlassen die Mitnehmer 30 die Innenhülse 29 und gelangen in Anlage mit der Innenhülsenoberkante 36. Bei der nächsten Betätigung wird die Betätigungskraft, die auf den Betätigungsdrücker 22 einwirkt, über die Innenhülse 29 auf die Mitnehmer 30 des Stößels 14 übertragen.

Ein (geringfügiges) Spiel zwischen Innenhülsenoberkante 36 und Mitnehmer 30 stellt einerseits sicher, daß die Mitnehmer 30 tatsächlich die Innenhülse 29 verlassen und ermöglicht andererseits erwünschte Hubwegdifferenzen der ersten und der zweiten Betätigung.

Um eine vorteilhafte große Widerverwertbarkeit von Teilen zu erreichen, kann der Betätigungsdrücker 22 durch Aufbringen einer radial wirkenden Kraft von dem Gehäuse 15 gelöst werden, in dem die Rastnasen 24 soweit in Richtung auf den Stößel 14 hineingedrückt werden, daß sie den Abschlußring 25 nicht mehr hintergreifen. Danach kann der Betätigungsdrüker 22 axial nach hinten abgezogen werden. Die zu diesem Zweck vorzugsweise an der Bodenfläche 35 des Betätigungsdrükers 22 befestigte Rückstellfeder 26 wird ebenfalls nach hinten abgezogen. Nun kann der Spritzenzylinder 11 aus dem Gehäuse 15 entfernt werden. Vorzugsweise wird gleichzeitig die Kappe 16 entfernt. Sofern der Stößel 14 nicht mit dem Kolben 12 verbunden ist, kann auch der Stößel 14 vom Spritzenzylinder herausgenommen werden und erneut verwendet werden. Ein ggf. mit einer Kappe 16 und Dichtelement 17 versehener Spritzenzylinder 11 mit dem darin befindlichen Kolben 12 und neuem, auszubringenden Medium kann nun in das Gehäuse 15 eingesetzt werden. Danach wird unter Erzeugen einer Vorspannung an der Rückstellfeder 26 der Betätigungsdrücker 22 wieder in den Schaft 33 des Gehäuses 15 eingesetzt. Sobald der Betätigungsdrücker 22 wieder durch den Abschlußring 25 gehalten wird, kann durch die Einführungsöffnung 34 des Betätiungsdrückers 22 hindurch der Stößel 14 wieder eingesetzt werden. Diese Vorgehensweise hat den Vorteil, daß der Stößel 14 nicht beim Wiederansetzen des Betätigungsdrückers 22 an dem Schaft 33 zu weit in Richtung des Ausbringungskanals 13 verschoben wird und so unbeabsichtigt ein Teil des auszubringenden Mediums schon vor der ersten Betätigung ausgebracht wird. Im Hinblick auf die Sicherung der Vorrichtung vor unbeabsichtigter Betätigung, kann es auch von Vorteil sein, den Stößel 14 erst unmittelbar vor einer weiteren Benutzung der Vorrichtung wieder durch die Einführungsöffnung 34 hindurch in das Gehäuse 15 und den Spritzenzylinder 11 hin einzusetzen, da ohne ihn ein unbeabsichtigtes Betätigen der Vorrichtung nicht möglich ist.

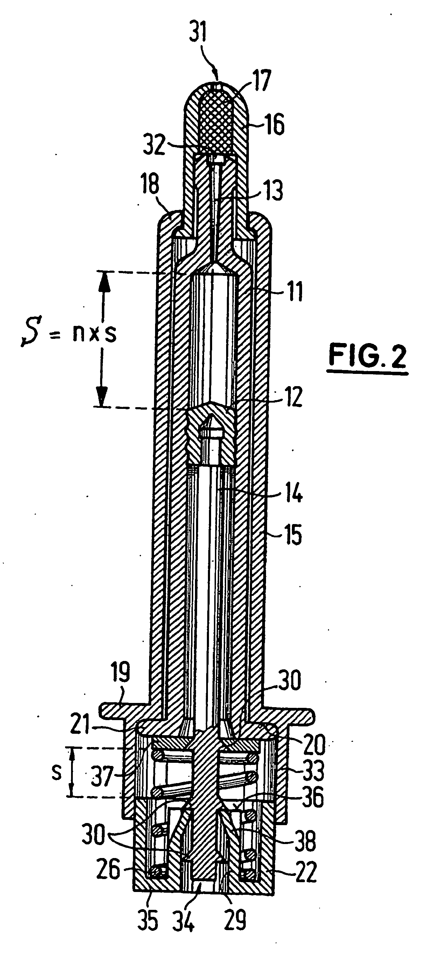

Die Fig. 2 zeigt ebenfalls einen Querschnitt durch eine Vorrichtung zum zerstäubten Ausbringen eines insbesondere flüssigen Mediums. Bei dieser Vorrichtung wird ebenfalls das Medium in dem Spritzenzylinder 11 bereitgehalten. Der Spritzenzylinder 11 mündet in dem Ausbringungskanal 13 mit seiner Mündung 32, die von dem in der Kappe 16 eingebrachten Dichtelement 17 zur Düse 31 hin verschlossen wird. Die Kappe 16 wird durch die Überwurfkante 18 des Gehäuses 15 am Spritzenzylinder gehalten. Der Spritzenzylinder 11 ist ebenfalls im Gehäuse 15 geführt. Auf der dem Ausbringungskanal 13 abgewandten Seite des Spritzenzylinders 11 ist der Kolben 12 axial verschiebbar angeordnet. Der Kolben 12 wird mittels des Stößels 14 und dem Betätigungsdrücker 22 manuell betätigt. Zur Aufbringung einer Kraft auf den Betätigungsdrücker 22 weist das Gehäuse 15 die Fingeranlage 19 auf. Der Spritzenzylinder 11 ist in dem Gehäuse 15 mit seiner Aufweitung 21 an der Anlagefläche 20 des Gehäuses 15 abgestützt. An der Aufweitung 21 des Spritzenzylinders 11 liegt die Rastscheibe 37 an. Zwischen Rastscheibe 37 - oder Aufweitung 21 - und der Bodenfläche 35 des Betätigungsdrückers 22 ist die Rückstellfeder 26, die für die Rückführung des Betätigungsdrükers 22 aus der Betätigungsendlage in die dargestellte Betätigungsausgangslage sorgt, angeordnet. Der Betätigungsdrücker 22 ist wiederum napfförmig ausgebildet und wird in dem Schaft 33 des Gehäuses 15 geführt. In der dargestellten Betätigungsausgangslage wird der Betätigungsdrücker 22 über nicht dargestellte Mittel am Schaft 33 so gehalten, daß er - vom Ausbringungskanal 13 her gesehen - nicht weiter nach hinten wegbewegt werden kann. Der napfförmige Betätigungsdrücker 22 weist auf seiner Bodenfläche 35 die Einführungsöffnung 34 auf, durch die hindurch der Stößel 14 in den Spritzenzylinder 11 so einführbar ist, daß er in Anlage an den Kolben 12 gelangt. In dieser Lage liegen die vordersten Mitnehmer 30 an der Rastscheibe 37 an. Die nächsten Mitnehmer 30, die von den vorhergehenden Mitnehmern 30 um die Länge s eines Teilhubes entfernt sind, werden von den Schubstegen 38 der Innenhülse 29 des Betätigungsdrückers 22 hintergriffen. Um unterschiedlich große Teilhübe zu ermöglichen, weisen die Mitnehmer 30 auch einen gegenüber der Länge s geringeren Abstand auf, der jedoch größer sein muß als s/2, damit nicht der übernächste Mitnehmer 30 hintergriffen wird. In diesem Fall gleiten die Schubstege 38 während eines Leerweges entlang des Stößels 14 bis die kraftschlüssige Verbindung zu dem Mitnehmer 30 des entsprechenden Ausbringungshubes entsteht. Die Innenhülse 29 umgibt die Einführungsöffnung 34 und ragt von der Bodenfläche 35 des Betätigungsdrückers 22 in Richtung auf den Spritzenzylinder 11 ab. Die Innenhülse 29 weist Schubstege 38 auf, die in den Bewegungsraum des Stößels 14 hineinragen, jedoch so elastisch sind, daß sie während des Rückhubes des Betätigungsdrückers 22 von der Betätigungsendlage in die Betätigungsausgangslage soweit radial nach außen gedrückt werden können, daß sie über die Mitnehmer 30 des Stößels 14 hinweg bewegt werden können und so den Bewegungsraum für die Relativbewegung zwischen Schaft 14 und Betätigungsdrücker 22 während des Rückhubes freigeben.

Wird der Betätigungsdrücker 22 betätigt, so muß zunächst eine solche Kraft aufgebracht werden, daß der vorderste Mitnehmer 30, der an der Rastscheibe 37 anliegt, die eine Rastkante bildet, die Rastscheibe 37 reversibel so spreizt, daß der Stößel 14 in den Spritzenzylinder 11 hineinbewegt werden kann. Ist diese Mindestbetätigungskraft überschritten, so wird der Schaft 14, von den Schubstegen 38 des Betätigungsdrückers 22 geschoben, weiter in den Spritzenzylinder 11 in Richtung auf den Ausbringungskanal 13 hineinbewegt und schiebt dabei den Kolben 12 des Spritzenzylinders 11 mit. Dies geschieht soweit, bis der Betätigungsdrücker 32 mit seiner dem Spritzenzylinder 11 zugewandten Oberkante an der Aufweitung 21 des Spritzenzylinders anliegt. In dieser Lage sind die ersten Mitnehmer 30 in den Spritzenzylinder 11 hineinbewegt, die zweiten Mitnehmer 30 liegen an der Rastscheibe 37 an, die Rückstellfeder 26 ist komprimiert. Wird der Betätigungsdrücker 22 nun losgelassen, so sorgt die Rückstellfeder 26 dafür, daß er wieder in die dargestellte Betätigungsausgangslage zurückbewegt wird. Dabei streifen die Schubstege 38 entlang dem Stößel 14, bis sie von der Vorderkante der dritten Mitnehmer 30 gespreizt werden und so über diese hinweg bewegt werden. Sobald die Schubstege 38 über die Mitnehmer 30 hinwegbewegt sind, legen sie sich wieder an den Stößel 14 an und hintergreifen die dritten Mitnehmer 30. Die zweiten Mitnehmer 30 dienen für den zweiten und im dargestellten Beispiel letzten Teilhub als Rastmittel, die das Aufbringen der erforderlichen Mindestbetätigungskraft für den weiteren Teilhub sicherstellen.

Im dargestellten Ausführungsbeispiel sind die Mitnehmer 30 als Verdickungen, d.h. als radiale Querschnittserweiterungen, des Stößels 14 ausgebildet. Der Stößel 14 weist im Bereich der Mitnehmer 30 einen kegelstumpfförmigen Querschnitt auf, wobei die Kegelspitze in Richtung des Kolbens 12 des Spritzenzylinders 11 zeigt. Die Schubstege 38 liegen zur Kraftübertragung auf den Stößel 14 an der Bodenfläche des Kegelstumpfes der Mitnehmer 30 an.

Um eine größere Anzahl von Teilhüben zu ermöglichen, ist es lediglich erforderlich, den Stößel 14 nach hinten zu verlängern und ihn mit weiteren Mitnehmern 30 zu versehen. Gleichzeitig muß der entsprechende Weg des Kolbens 12 im Spritzenzylinder 11 vorhanden sein.

## Patentansprüche

1. Vorrichtung zum ggf. zerstäubten Ausbringen eines flüssigen Mediums, wobei das Medium aus einem Spritzenzylinder (11), in dem ein Kolben (12) über einen Betätigungsweg (S) zwischen einer Betätigungsausgangslage und einer Betätigungsendlage verschiebbar gelagert ist, über einen Ausbringungskanal (13) ausbringbar ist, wobei der Spritzenzylinder (11) und der Kolben (12) manuell in wenigstens zwei voneinander abgegrenzten, den Betätigungsweg (S) unterteilenden Teilhüben (s) zur Ausgabe von wenigstens zwei gleichen Teilchargen mittels eines Betätigungsdrückers (22) manuell relativ zueinander bewegbar sind, wobei die jeweiligen Betätigungswege für die Teilhübe (s) auf unterschiedliche Größen abgegrenzt sind, **dadurch gekennzeichnet, dass** ein erster Teilhub größer ist als ein zweiter Teilhub, um ein Öffnen eines Dichtelements (17) im Bereich des Ausbringungskanales (13) zu gestatten.

2. Vorrichtung nach Anspruch 1, **dadurch gekennzeichnet, daß** eine Rückstellfeder (26) so angeordnet ist, daß der Betätigungsdrücker (22)nach Ausführung eines Teilhubes (s) selbsttätig in die Betätigungsausgangslage zurückgeführt wird.

3. Vorrichtung nach Anspruch 1 oder 2, **dadurch gekennzeichnet, daß** an einem den Betätigungsdrücker (22) mit dem Kolben (12) verbindenden Stößel (14) in den Teilhüben (s) entsprechendem Abstand Mitnehmer (30) angeformt sind, wobei die Mitnehmer (30) lediglich in Richtung des Ausbringungshubes eine kraftschlüssige Verbindung zwischen Betätigungsdrücker (22) und Stößel (14) herstellen und der Betätigungsweg (s) des Betätigungsdrückers (22) auf das für den größten Ausbringungshub erforderliche Maß begrenzt ist.

4. Vorrichtung nach Anspruch 3, **dadurch gekennzeichnet, daß** an dem Stößel (14) Rastmittel (27) angeformt sind, die in Betätigungsausgangslage an einer Rastkante (28) anliegen und die durch Überwinden einer Mindestbetätigungskraft überdrückbar sind, wobei vorzugsweise die Mitnehmer (30) so ausgebildet sind, daß der Mitnehmer (30) eines Ausbringungshubes als Rastmittel eines nachfolgenden Ausbringungshubes dient.

5. Vorrichtung nach einem der Ansprüche 3 oder 4, **dadurch gekennzeichnet, daß** die Mitnehmer (30) als Verdickungen des Stößels und insbesondere aus kegelstumpfförmigen Abschnitten des Stößels (14) gebildet sind, deren imaginäre Kegelspitze in Richtung des Kolbens (12) weist.

6. Vorrichtung nach einem der Ansprüche 1 bis 5, **dadurch gekennzeichnet, daß** der Spritzenzylinder (11) in einem Gehäuse (15) gehalten ist, wobei das Gehäuse (15) vorzugsweise angeformte Fingerauflagen (19) für wenigstens einen Finger aufweist.

7. Vorrichtung nach Anspruch 6, **dadurch gekennzeichnet, daß** der Betätigungsdrücker (22) im Gehäuse (15) geführt und insbesondere lösbar an dem Gehäuse (15) angeordnet ist.

8. Vorrichtung nach einem der Ansprüche 1 bis 7, **dadurch gekennzeichnet, daß** der Ausbringungskanal (13) in einer Kappe (16) mündet, wobei die Kappe (16) eine Zerstäuberdüse (31) aufweist.

9. Vorrichtung nach Anspruch 8, **dadurch gekennzeichnet, daß** der Strömungsweg zwischen Ausbringungskanal (13) und Zerstäuberdüse (31) über ein ein Auslaßventil bildendes Dichtelement (17) verschlossen ist, das den Strömungsweg aufgrund des Mediendrucks während der Betätigung des Betätigungsdrükers (22) reversiebel oder irreversibel bei der ersten Betätigung des Betätigungsdrückers (22) freigibt.

10. Vorrichtung nach einem der vorhergehenden Ansprüche, **dadurch gekennzeichnet, daß** zumindest der Spritzenzylinder (11) mit seinem Kolben (12) austauschbar im Gehäuse (15) gehalten ist, wobei vorzugsweise zusätzlich wenigstens eines der folgenden Elemente: Stößel (14), Kappe (16) und Dichtelement (17) austauschbar ist.

## Claims

1. Apparatus for if necessary atomized dispensing of a liquid medium, the medium being dispensable via a dispensing channel (13) from a syringe cylinder (11) in which a piston (12) is mounted movably over an actuating path (S) between an actuation starting position and an actuation end position, the syringe cylinder (11) and the piston (12) being manually movable relative to one another in at least two partial strokes (s) delimited from one another and subdividing the actuating path (S) for dispensing of at least two equal partial batches by means of an actuating pusher (22), where the respective actuating paths for the partial strokes (s) are delimited to different sizes, **characterized in that** a first partial stroke is larger than a second partial stroke in order to allow opening of a sealing element (17) in the area of the dispensing channel (13).

2. Apparatus according to Claim 1, **characterized in that** a reset spring (26) is arranged such that the actuating pusher (22) is automatically returned to the actuation starting position after performance of a partial stroke (s).

3. Apparatus according to Claim 1 or 2, **characterized in that** drivers (30) are integrally moulded on a plunger (14) connecting the actuating pusher (22) to the piston (12) at a distance corresponding to the partial strokes (s), the drivers (30) making a non-positive connection between the actuating pusher (22) and the plunger (14) only in the direction of the dispensing stroke, and the actuating path (s) of the actuating pusher (22) being limited to the dimension required for the largest dispensing stroke.

4. Apparatus according to Claim 3, **characterized in that** engaging means (27) are integrally moulded on the plunger (14) which contact an engaging edge (28) in the actuation starting position and which can be pushed past by overcoming a minimum actuating force, the drivers (30) preferably being designed such that the driver (30) of one dispensing stroke acts as the engaging means of a subsequent dispensing stroke.

5. Apparatus according to one of Claims 3 or 4, **characterized in that** the drivers (30) are designed as thickened portions of the plunger and in particular as frustum-shaped sections of the plunger (14), of which the imaginary cone tip points in the direction of the piston (12).

6. Apparatus according to one of Claims 1 to 5, **characterized in that** the syringe cylinder (11) is held in a housing (15), the housing (15) preferably having integrally moulded finger rests (19) for at least one finger.

7. Apparatus according to Claim 6, **characterized in that** the actuating pusher (22) is guided in the housing (15) and in particular is arranged detachably on the housing (15).

8. Apparatus according to one of Claims 1 to 7, **characterized in that** the dispensing channel (13) opens into a cap (16), the cap (16) having an atomizing nozzle (31).

9. Apparatus according to Claim 8, **characterized in that** the flow path between the dispensing channel (13) and the atomizing nozzle (31) is closed by a sealing element (17) forming an outlet valve and releasing the flow path reversibly or irreversibly during the first actuation of the actuating pusher (22) due to the media pressure during actuation of the actuating pusher (22)

10. Apparatus according to one of the preceding claims, **characterized in that** at least the syringe cylinder (11) with its piston (12) is exchangeably held in a housing (15), where preferably at least one of the following elements of plunger (14), cap (16) and sealing element (17), is additionally exchangeable.

## Revendications

1. Dispositif pour délivrer, notamment pour pulvériser un fluide liquide, sachant que le fluide peut être délivré par un conduit de décharge (13) à partir d'un cylindre de seringue (11) dans lequel est logé un piston (12) pouvant se déplacer le long d'une trajectoire d'actionnement (S) entre une position initiale d'actionnement et une position finale d'actionnement, sachant que le cylindre de seringue (11) et le piston (12) peuvent être déplacés manuellement l'un par rapport à l'autre au moyen d'un poussoir d'actionnement (22) sur au moins deux courses partielles (s) délimitées l'une par rapport à l'autre et subdivisant la trajectoire d'actionnement (S) afin de délivrer au moins deux doses partielles égales, sachant que les trajectoires d'actionnement respectives pour les courses partielles (s) sont limitées à des mesures différentes, **caractérisé en ce qu'**une première course partielle est supérieure à une seconde course partielle afin de permettre l'ouverture d'un élément d'étanchéité (17) dans la zone du conduit de décharge (13).

2. Dispositif selon la revendication 1, **caractérisé en ce qu'**un ressort de rappel (26) est disposé de telle manière que le poussoir d'actionnement (22) est ramené automatiquement dans la position initiale d'actionnement après exécution d'une course partielle (s).

3. Dispositif selon la revendication 1 ou 2, **caractérisé en ce que** sur une tige-poussoir (14) reliant le poussoir d'actionnement (22) au piston (12) sont formés des entraîneurs (30) avec un écart correspondant aux courses partielles (s), sachant que les entraîneurs (30) créent un liaison à force entre le poussoir d'actionnement (22) et la tige-poussoir (14) uniquement en direction de la course de décharge, et que la trajectoire d'actionnement (s) du poussoir d'actionnement (22) est limitée à la mesure requise pour la course de décharge maximale.

4. Dispositif selon la revendication 3, **caractérisé en ce que** des moyens de crantage (27) sont formés sur la tige-poussoir (14) et qui s'appuient sur une arête d'arrêt (28) dans la position initiale d'actionnement et qui peuvent être franchis en surmontant une force minimale d'actionnement, sachant que de préférence les entraîneurs (30) sont réalisés de telle manière que l'entraîneur (30) d'une course de décharge puisse servir de moyen de crantage pour une course de décharge ultérieure.

5. Dispositif selon l'une des revendications 3 ou 4, **caractérisé en ce que** les entraîneurs (30) sont réalisés en tant que renflements de la tige-poussoir et notamment en tant que sections de forme tronconique de la tige-poussoir (14) dont la pointe imaginaire du cône est orientée en direction du piston (12).

6. Dispositif selon l'une des revendications 1 à 5, **caractérisé en ce que** le cylindre de seringue (11) est maintenu dans un boîtier (15), sachant que le boîtier (15) présente des appuis pour les doigts (19), formés de préférence par moulage, pour au moins un doigt.

7. Dispositif selon la revendication 6, **caractérisé en ce que** le poussoir d'actionnement (22) est guidé dans le boîtier (15) et qu'il est disposé sur le boîtier (15) de manière en particulier détachable.

8. Dispositif selon l'une des revendications 1 à 7, **caractérisé en ce que** le conduit de décharge (13) débouche dans un capuchon (16), sachant que le capuchon (16) présente une buse de pulvérisation (31).

9. Dispositif selon la revendication 8, **caractérisé en ce que** la voie d'écoulement entre le conduit de décharge (13) et la buse de pulvérisation (31) est fermée par un élément d'étanchéité (17) formant une soupape de sortie qui débloque la voie d'écoulement de manière réversible ou irréversible pendant l'actionnement du poussoir d'actionnement (22) sous l'effet de la pression du fluide lors du premier actionnement du poussoir d'actionnement (22).

10. Dispositif selon l'une des revendications précédentes, **caractérisé en ce qu'**au moins le cylindre de seringue (11) avec son piston (12) est maintenu de manière remplaçable dans le boîtier (15), sachant que de préférence en outre au moins un des éléments suivants : la tige-poussoir (14), le capuchon (16) et l'élément d'étanchéité (17) est remplaçable.
